# EUROPEAN PATENT APPLICATION

(11) **EP 1 712 596 A2**
(43) Date of publication of application: **18.10.2006**
(21) Application number: 06007337.6
(22) Date of filing: 06.04.2006
(51) Int. Cl.: C09B 67/08, A61K 8/365

(54) **Surface-treated pigment and process for producing the same**

(30) Priority: 06.04.2005 MY 0501537
(71) Applicant: Malaysian Palm Oil Board, 43000 Kajang, Selangor Darul Ehsan (MY)
(72) Inventor: Zahariah, Ismail Malaysian Palm Oil Board, 43000 Kajang Selangor Darul Ehsan (MY); Salmiah, Ahmad Malaysian Palm Oil Board, 43000 Kajang Selangor Darul Ehsan (MY); Parthiban, Siwayanan Malaysian Palm Oil Board, 43000 Kajang Selangor Darul Ehsan (MY); Hazimah Abu, Hassan Malaysian Palm Oil Board, 43000 Kajang Selangor Darul Ehsan (MY); Rosnah, Ismail Malaysian Palm Oil Board, 43000 Kajang Selangor Darul Ehsan (MY); Aishah Ahmad Malaysian Palm Oil Board, 43000 Kajang Selangor Darul Ehsan (MY); Zafarizal Aldrin A. Hasan Malaysian Palm Oil Board, 43000 Kajang Selangor Darul Ehsan (MY); Suriati, Hashim Malaysian Palm Oil Board, 43000 Kajang Selangor Darul Ehsan (MY); Yusrabbil Amiyati, Yusof Malaysian Palm Oil Board, 43000 Kajang Selangor Darul Ehsan (MY); Zulina Abd. Maurad Malaysian Palm Oil Board, 43000 Kajang Selangor Darul Ehsan (MY); Tuan Noor M. T. Ismail Malaysian Palm Oil Board, 43000 Kajang Selangor Darul Ehsan (MY); Rubaah, Masri Malaysian Palm Oil Board, 43000 Kajang Selangor Darul Ehsan (MY); Rozana Abu, Bakar Malaysian Palm Oil Board, 43000 Kajang Selangor Darul Ehsan (MY); Haliza Abd., Aziz Malaysian Palm Oil Board, 43000 Kajang Selangor Darul Ehsan (MY); Hoong Seng, Soi Malaysian Palm Oil Board, 43000 Kajang Selangor Darul Ehsan (MY); Norashikin, Ahmad Malaysian Palm Oil Board, 43000 Kajang Selangor Darul Ehsan (MY); Norin Zamiah K., Shaari Malaysian Palm Oil Board, 43000 Kajang Selangor Darul Ehsan (MY); Mohd Norhisham, Sattar Malaysian Palm Oil Board, 43000 Kajang Selangor Darul Ehsan (MY)
(74) Representative: Urizar Anasagasti, José Antonio

(57) **Abstract**

Pigments are used to impart color in finished products. The performances of pigments can be improved by coating with suitable materials rendering suitable properties. For cosmetic applications the pigments are usually surface treated with hydrophobizing material rendering the pigments hydrophobic. This invention relates to the use of polyhydroxy fatty acid preferably dihydroxysteadc acid (DHSA) and/or metallic salts of dihydroxystearic acid (Metallic DHSA) to coat pigments in particular pigments to be used in color cosmetic formulations. DHSA is produced either from crude or refined oleic acid or via chemical transformation or biotransformation of suitable substrates, used either in crude or refined form and the metallic salts are produced either using crude or refined DHSA, DHSA and Metallic DHSA are used to coat pigments and when these coated pigments are incorporated into finished products formulations such as cosmetic formulations, enhanced performances are observed. In color cosmetics the enhanced performances include enhancing skin adhesion, better spreadability, increasing strength of the stick, increasing apparent size and increased hydrophobicity of make-up cosmetics such as lipsticks, foundations, mascaras, eye shadows etc.

## Description

### Field of Invention

The present invention relates to a pigment material of which the surfaces are coated with a coating comprising of at least one polyhydroxy fatty acid or derivative thereof, wherein said polyhydroxy fatty acid is preferably dihydroxystearic acid (DHSA) or metallic dihydroxystearic acid (metallic DHSA). The present invention also relates to a process for coating the afore-mentioned pigment material.

### Background of the Invention

Make-up cosmetics, such as lipstick, foundation, mascara and powders are commonly prepared with main constituents such as pigments, extender pigments, binders, emulsifiers, emollient, rheological additives and other additives and are used to enhance personal attractiveness and to improve self-esteem

In the past, uncoated pigments may cause a tendency to induce oxidation reaction, incompatibility or insufficient wettability with several oils, and impair coloring performance.
Another problem is the difficulty in shading the colors and the fact that uncoated pigments may cause Toatation and/settling' in fluid pigmented emulsions like in liquid foundation make-up.

The so-called 'treated pigment' is to improve the wear and long lasting characteristics therefore, the surface of pigments used in the cosmetics were coated with silicone, metal soap, fluorocompounds, hydrogenated lecithin, fatty acid triglycerides, and others to render such materials hydrophobic ("hyrophobidization").

In US Patent No. 5.578,311 on surface-coating of pigments and extender pigments with fluorine compound claimed to improve good spreadibility on the skin, desirable feels when use, for example, a moist and refreshing feel.

U.S. Patent No. 5.368,639 on surface-coating of pigments and extender pigments with organosilicone compounds provides a good adhesion to the skin, very smooth feel, and ability to permit the color pigment of fine particles to spread well. The treated pigment finds its use as a component of high quality cosmetics such as powder foundation, liquid foundation, rouge, and eye shadow.

U.S. Pat. No. 5, 174,996 on surface-coating of pigments and extender pigments with oxidized polyethylene compounds is to prevent from settling and migration of pigments and other raw materials suspended in nail enamel.

U.S. Pat. No. 5, 326,392 on surface-coating of pigments and extender pigments with lauroyl lysine is useful for color cosmetics especially, such as eye shadow

U.S. Pat. No. 4, 863,800 on surface-coating of pigments and extender pigments with a saturated fatty acid triglycerides is used in cosmetics that has strong water repellency, smooth feel, and adheres well to the skin.

U.S. Pat. No. 4, 863,800 on surface-coating of pigments and extender pigments with hydrogenated lecithin or with the reaction product of hydrogenated lecithin and a metal salt exhibits excellent protection for the skin, resistance to wear, and good water repellency.

U.S. Pat. No. 4, 640,943 on surface-coating of pigments and extender pigments with an N-acylated basic amino acid is to improve the compatibility of the filler materials with a variety of different formulations

### Summary of the Invention

It is accordingly an object of the present invention to provide a surface-treated pigment material of which the surfaces are coated with a coating comprising of at least one polyhydroxy fatty acid or derivative thereof, wherein said polyhydroxy fatty acid is obtainable from hydroxylating of a fatty acid having at least 12 carbon atoms.

It is another object of the present invention to provide a surface-treated pigment with improved characteristics for use in the formulation of variety of different products.

Still, another object of the present invention is to provide a surface-treated pigment suitable for blending with cosmetics.

A further object of the present invention to improve the properties of finished products in terms of such as smooth feel, good adhesion to the skin, good spreadability, increased strength of the stick, voluminizing effect and increased hydropobicity for color cosmetic.

Yet, an object of the present invention to provide a process for producing afore-said surface-treated pigment and a cosmetic product, which contains said surface-treated pigment.

These objects and other objects of the present invention will become more apparent from the following description.

In one embodiment of the present invention, a surface-treated pigment material comprising of pigment material of which the surfaces are coated with a coating comprising of at least one polyhydroxy fatty acid or derivative thereof, wherein said polyhydroxy fatty acid is obtainable from hydroxylating of a fatty acid having at least 12 carbon atoms.

The polyhydroxy fatty acid is preferably dihydroxystearic acid (DHSA) or metallic salts of dihydroxystearic acid (metallic DHSA) or combination thereof of about 0.001% to 50% by weight based on the amount of pigment material to be treated. The DHSA and metallic DHSA may be derived either via chemical transformation or biotransformation of crude or refined fatty acid contained in natural fats, oils or waxes.

According to the present invention, coating of pigment with DHSA or metallic DHSA will result in the finished products having the ability to impart hydrophobicity, smooth feel, improve adhesion to the skin, and is free from agglomeration because of uniform coating on each pigment. The coated pigments of the present invention is also stable in cosmetic formulations, improve coloring effect, dispersion of pigment, increase strength of the stick, improve the curling effect of mascara, reduce the drying time, increase in apparent size of lashes, has a thickening property in polar as well as non polar oils, reduces oil absorbing power in polar and non-polar oils.

In another embodiment of the present invention, a process for coating said pigment material comprising the steps of:
a. dissolving a polyhydroxy fatty acid into an alcoholic solution, wherein said polyhydroxy fatty acid is preferably dihydroxystearic acid (DHSA), metallic salts of dihydroxystearic acid (metallic DHSA) or combination thereof.
b. neutralizing said dissolved polyhydroxy fatty acid using an alkali solution preferably NaOH;
c. dissolving any one or combination of metal chloride, metal hydroxides or metal oxides into an alcoholic solution preferably 50% of hydro-alcoholic solution;
d. dispersing pigment material into said metal solution from step (c);
e. adding neutralized polyhydroxy fatty acid from step (b) into metal solution from step (d);
f. filtering the resultant product from step (e) to obtain the coated pigment; and
g. drying of said coated pigment.

The process may further comprising a step of washing said coated pigment with an alcoholic solution prior to drying.

The process may further comprising a step of heating up the neutralized polyhydroxy fatty acid prior to step (e).

### Detailed Description of the Preferred Embodiments

The present invention relates to a surface-treated pigment material comprising of pigment material of which the surfaces are coated with a coating comprising of at least one polyhydroxy fatty acid or derivative thereof, wherein said polyhydroxy fatty acid is obtainable from hydroxylating of a fatty acid having at least 12 carbon atoms.

The polyhydroxy fatty acid according to the present invention is preferably dihydroxystearic acid (DHSA) or metallic salts of dihydroxystearic acid (metallic DHSA) or combination thereof of about 0.001% to 50% by weight based on the amount of pigment material to be treated. The DHSA and metallic DHSA may be derived either via chemical transformation or biotransformation of crude or refined fatty acid contained in natural fats, oils or waxes.

More preferably, the DHSA used in the present invention is obtainable by a process according to Malaysia Patent Application No. 20041450, which thereinafter incorporated as a reference.

The present invention also provides a process for producing a coated pigment material mentioned above.

First, the polyhydroxy fatty acid preferably dihydroxystearic acid (DHSA) is dissolved into an alcoholic solution, preferably 50% hydro-alcoholic solution. The amount of DHSA to use may vary from 0.001% to 50% usually in the range of 10% of the pigment. DHSA is then neutralized with an alkali solution, preferably an equimolar amount of NaOH. The final pH of the solution should be around 8-9. DHSA sodium salt is completely soluble in the hydro alcoholic solution with the help of gentle heating (<85°C).

Meanwhife, another hydro-alcoholic solution (50%) is used to dissolve the stoichiometric amount (calculated based on DHSA) of any one or combination of metal chlorides, hydroxides or oxides. In the preferred embodiment of the present invention, metal chlorides are used, such as ZnCl₂, Al₂Cl₃, MgCl₂, CaC1₂ or other chlorides may also be used.

Then, the resulting metal chlorides solution is mixed thoroughly with the pigment material to be treated. The pigment materials that can be coated include titanium dioxide, red iron oxide, yellow iron oxide and black iron oxide but other pigments can also be coated.

The prepared warm solution of neutralized DHSA is subsequently slowly added, under stirring, into the hydro-alcoholic pigment suspension containing metal chloride. The solution is left to stand to allow precipitation of coated pigment. Metal dihydroxystearate coated pigment is then filtered and successively washed with a hyro-alcoholic solution (50%) to eliminate all traces of NaCl₂. The coated pigment is then dried and ready to be used in finished product formulations such as cosmetic formulations.

The present invention will hereafter be described in detail with reference to the following examples which are included herein solely as an illustrative aid to provide a more complete understanding of the present invention and the product formed thereby. The examples do not limit the scope of the present invention disclosed and claimed herein in any fashion.

### Example 1

### Using DHSA to coat pigment

10g DHSA was heated in 50% hydro-alcoholic solution to dissolve. It was then neutralized by an equimolar amount of NaOH. The weight of CaCl₂ was calculated based on the same mole ratio of DHSA. In another vessel, CaC1₂ was heated in 50% hydro-alcoholic to dissolve. The mixture was adjusted to pH 3. 90 g of red iron oxide was suspended into calcium chloride solution and thoroughly mixed for a perfect wetting. Then sodium DHSA solution was slowly added. Finally, the pH of the suspension was adjusted to 6, if necessary. The suspension was allowed to sit and the precipitate formed was decanted, washed, filtered and finally air-dried. This precipitation is known as Ca DHSA red iron oxide.

### Example 2

### Using Metallic DHSA to coat pigment

Other metallic DHSA coated pigment may be prepared in a similar manner of Example 1.

### Example 3

### Using Metallic DHSA coated pigment in mascara

The same procedure as in example 1 was repeated except that the calcium chloride and red iron oxide were replaced by zinc chloride and black iron oxide. The Zn DHSA black iron oxide was added in the mascara formulae as illustrated below:-

A typical water/oil liquid mascara product was prepared using the weight percent of the various ingredients as indicated in the table of Formula for Example 3.
1. Phase C was prepared. C1 was added to phase C and swelled for I hour followed with the addition of C2. The mixture was then heated and homogenized at 800C -850C for 5 minutes.
2. Phase A was heated to 850C. Phase Al was added into phase A while homogenizing for 5 minutes to verify the perfect dispersion of pigments.
3. Mixture of A and Al was added slowly to phase B which was heated to 850C while homogenizing.
4. Phase C consists of C1 and C2 was added to the mixture of phase A+A1 +13 while homogenizing for 5 minutes. It was then cooled under room temperature under slow mixing condition.

| **Formula For Example 3** | | |
|---|---|---|
| Phase | Component | Weight % |
| A | Hydrogenated polydecene | 1.0 |
| | Medium chain triglycerides | 1,0 |
| | Phenonip | 1.2 |
| | Tocopherol acetate | 0.3 |
| | DHSA | 3.0 |
| | Beeswax | 4.0 |
| | Candellila wax | 4.0 |
| | Camauba wax | 4.0 |
| | Brij 721 | 3.0 |
| A1 | Zn-DHSA black pigment | 10.0 |
| B | Aqua | 15.0 |
| | Triethanolamine | 2.2 |
| C | Aqua | 51.3 |
| | Disodium EDTA | 0.1 |
| C1 | Magnesium Aluminium Silicate | 1.7 |
| C2 | Natrosol 250 | 1,2 |

For purposes of comparison, DHSA and Zn-DHSA black pigment were replaced with stearic acid and uncoated black pigment.

### Evaluation method

10 skilled panelists evaluated the effects of treated and untreated pigment in mascara. The resultant treated pigment improves the curling effect, reduce the drying time, increase in apparent size of lashes as compared to the uncoated pigment.

### Example 4

### Using Metallic DHSA coated pigment in foundation

The same procedure as in Example 1 was repeated except that the red iron oxide was replaced by black iron oxide. The same procedure was repeated as in example 1 except that the red iron oxide was replaced by yellow iron oxide. The same procedure was repeated as in example 1 except that the red iron oxide was replaced by titanium dioxide.

The Ca DHSA red iron oxide, Ca DHSA yellow iron oxide, and Ca DHSA titanium dioxide were added in the foundation formulae as in Formula for Example 4 shown below:

| **Formula for Example 4** | | |
|---|---|---|
| **Phase** | **Component** | **Weight %** |
| A | Aqua | 36.40 |
| | Disodium EDTA | 0.05 |
| | Betaine | 2.00 |
| | Propylene glycol | 2.00 |
| | Glycerine | 6.00 |
| Al | Magnesium aluminium silicate | 0.70 |
| A2 | Microcrystal line Cellulose | 0.50 |
| | Cellulose gum | |
| B | Hydogenated polydecene | 6.0 |
| | Butylene glycol | 7.50 |
| | Dicapylate/dicaprate Triglyceride | 6.00 |
| | Isopropyl palmitate | 4.00 |
| | Beeswax | 4.00 |
| | Glyceryl stearate | 2.00 |
| | Polyhyroxystearic Acid | 0.50 |
| | Ceteth-20 | 2.00 |
| | Propylparaben | 0.15 |
| | Methyparaben | 0.30 |
| | BHT | 0.05 |
| | | |
| B1 | C.l 77492* | 0.70 |
| | C.l 77491 * | 11.50 |
| | C.l 77891 * | 3.00 |
| C | Aluminium starch | 3.00 |
| | Ocenylsuccinate | 0.20 |
| D | Aqua | 0.15 |
| | Quartenium -15 | 0.02 |
| E | Perfume | 0-15 |
| * pigment coated with DHSA 10% | | |

1. A1 and A2 were disperse in phase A while mixing after each addition, then heated at 75°C.
2. Phase B was heated to 75°C and then phase B1 was, added successively while homogenizing till complete dispersion of pigments.
3. Finally, the mixture of A+Al+A2 was added into B+B1 while homogenizing. The product was cooled to 40°C and C, D, and E were added successively while mixing after each addition.
4. For purposes of comparison, Ca DHSA red iron oxide, Ca DHSA yellow iron oxide, and Ca DHSA titanium dioxide were replaced with uncoated pigment.

### Evaluation method

10 skilled panelists evaluated the effects of coated and uncoated pigment by applying the foundation in which half of the face with coated pigment and another half with uncoated pigment. It was found that the foundation made from coated pigment was much easier to spread onto the skin when compared to uncoated pigment.

Based on the appearance of the product, the resultant coated pigment improves coloring effect compared to uncoated pigment in which less quantity of coated pigment is needed than with usual system.

### Example 5

### Using Metallic DHSA coated pigment in lipstick

The same procedure as in example 1 was repeated except that the calcium chloride was replaced by magnesium chloride. The same procedure was repeated by using other pigment such as yellow iron oxide, black iron oxide and titanium dioxide. The Mg DHSA red iron oxide, Mg DHSA yellow iron oxide, Mg DHSA black iron oxide and Mg DHSA titanium dioxide were added in the lipstick formula for example 5 as shown below.

| **Formula for Example 5** | | |
|---|---|---|
| **Phase** | **Component** | **Weight %** |
| A | Hydrogenated polydecene | 34.50 |
| | C.1 1 77492* | 0.95 |
| | C.1 77491* | 0.70 |
| | CL 77499* | 0.65 |
| | C. 177891 | 3.95 |
| | | |
| B | Ozokerite | 5.5 |
| | Candellila wax | 5.0 |
| | Beeswax | 8.0 |
| | Carnauba wax | 1,5 |
| | Hydrogenated Palm Kernel olein | 6.5 |
| | Anhydrous lanolin | 7,0 |
| | Hydogenated polydecene | 6.0 |
| | Isopropyl Myristate | 7.50 |
| | Isopropyl palmitate | 5.00 |
| | Cetyl alcohol | 0.5 |
| | Phenyl trimethicone | 3.0 |
| B1 | Tocopheryl acetate | 0.5 |
| | Plastic powder | 3.0 |
| * pigment coated with DHSA 10% | | |

1. Phase A was melted to 90°C while homogenizing.
2. The phase B was heated to 90°C and transferred to phase B1 while stirring. The mixture was cooled to 80°C and poured to lipstick moulds.
3. The mould was cool to 5°C for 30 minutes.
4. The lipstick was detached and put into casing.
5. For purposes of comparison, Mg DHSA red iron oxide,'Mg DHSA yellow iron oxide, Mg DHSA black iron oxide and Mg DHSA titanium dioxide were replaced with uncoated pigment.

### Evaluation method

10 skilled panelists evaluated the effects of coated and uncoated pigment by applying the lipstick onto their lips. It was found that the spreading effect of lipsticks with the coated pigment were much easier to apply compared to lipsticks with uncoated pigment. The breaking point test confirmed that the lipsticks with coated pigment were stronger.

It should be understood that the foregoing description is only illustrative of the present invention. Various alternatives and modifications can be devised by those skilled in the art without departing from the invention. Accordingly, the present invention is intended to embrace all such alternatives, modifications and variances which fall withon of the appended claims.

## Claims

1. A surface-treated pigment material comprising of pigment material of which the surfaces are coated with a coating comprising of at least one polyhydroxy fatty acid or derivative thereof, wherein said polyhydroxy fatty acid is obtainable from hydroxylating of a fatty acid having at least 12 carbon atoms.

2. A surface-treated pigment material according to Claim 1, wherein said polyhydroxy fatty acid or derivative thereof is about 0.001 % to 50% by weight based on the amount of pigment material to be treated.

3. A surface-treated pigment material according to Claim 1 or 2, wherein said polyhydroxy fatty acid is dihydroxystearic acid (DHSA) or metallic salts of dihydroxystearic acid (metallic DHSA) or combination thereof.

4. A process for coating a pigment material comprising the steps of:
a. dissolving a polyhydroxy fatty acid into an alcoholic solution;
b. neutralizing said dissolved polyhydroxy fatty acid using an alkali solution;
c. dissolving any one or combination of metal chloride, metal hydroxides or metal oxides into an alcoholic solution;
d. dispersing pigment material into said metal solution from step (c);
e. adding neutralized polyhydroxy fatty acid from step (b) into metal solution from step (d);
f. filtering the resultant product from step (e) to obtain the coated pigment; and
g. drying of said coated pigment.

5. A process for coating a pigment material according to Claim 7, wherein said process further comprising a step of washing said coated pigment with an alcoholic solution prior to drying.

6. A process for coating a pigment material according to Claim 7, wherein said polyhydroxy fatty acid is dihydroxystearic acid (DHSA) or metallic salts of dihydroxystearic acid (metallic DHSA) or combination thereof.

7. A process for coating a pigment material according to Claim 7, wherein said alcoholic solution is 50% of hydro-alcoholic solution,

8. A process for coating a pigment material according to Claim 7 or 8, wherein said alkali solution is preferably NaOH.

9. A process for coating a pigment material according to Claim 7, wherein said process further comprising a step of heating up the neutralized polyhydroxy fatty acid prior to step (e).
